## Europäisches Patentamt
## European Patent Office
## Office européen des brevets

(19)

(11) Veröffentlichungsnummer: **0 021 433**
**B1**

(12)
# EUROPÄISCHE PATENTSCHRIFT

(45) Veröffentlichungstag der Patentschrift:
**12.01.83**

(51) Int. Cl.³: **C 07 C 51/487, C 11 C 1/10**

(21) Anmeldenummer: **80103622.9**

(22) Anmeldetag: **26.06.80**

(54) **Verfahren zur Reinigung von Fettsäure- oder Fettsäureestergemischen.**

(30) Priorität: **02.07.79 DE 2926635**

(43) Veröffentlichungstag der Anmeldung:
**07.01.81 Patentblatt 81/1**

(45) Bekanntmachung des Hinweises auf die Patenterteilung:
**12.01.83 Patentblatt 83/2**

(84) Benannte Vertragsstaaten:
**AT BE CH DE FR GB IT LI NL SE**

(56) Entgegenhaltungen:
**CH-A-278 933**
**FR-A-2 210 601**
**FR-A-2 236 830**
**GB-A-639 550**
**US-A-2 341 239**

(73) Patentinhaber: **Henkel Kommanditgesellschaft auf Aktien, Postfach 1100 Henkelstrasse 67, D-4000 Düsseldorf 1 (DE)**

(72) Erfinder: **Hartmann, Helmut, Dr., Kolpingstrasse 4, D-4018 Langfeld (DE)**
Erfinder: **Hentschel, Karl, Boschstrasse 40, D-4000 Düsseldorf 13 (DE)**

Verfahren zur Reinigung von Fettsäure- oder Fettsäureestergemischen

Die Erfindung betrifft ein Verfahren, Polyenfettsäuren wie Linol- und Linolensäure aus Gemischen von Fettsäuren oder Fettsäureestern zu entfernen, die gleichzeitig Monoenfettsäuren enthalten und vorzugsweise hauptsächlich aus solchen Monoenfettsäuren bestehen. Insbesondere betrifft die Erfindung ein verbessertes Verfahren zur Herstellung von technischer Ölsäure (Olein) mit einem niedrigen Linolsäuregehalt.

Die nach verschiedenen Verfahren gewinnbaren flüssigen Anteile von Fettsäuregemischen tierischen oder pflanzlichen Ursprungs, die als Olein oder technische Ölsäure bezeichnet werden, enthalten als Hauptbestandteil Ölsäure, in manchen Fällen auch Palmitölsäure sowie neben gesättigten Fettsäuren mehrfach ungesättigte Fettsäuren, insbesondere Linol- und Linolensäure in unterschiedlichen Mengen. Bei verschiedenen Anwendungsgebieten der technischen Ölsäure ist ihr Gehalt an Polyenfettsäuren unerwünscht. Man ist hier gezwungen, die mehrfach ungesättigten Anteile aus den ölsäurereichen Fettsäuregemischen durch geeignete Massnahmen zu entfernen.

Es sind bereits mehrere Verfahren bekannt geworden, bei denen man die in der technischen Ölsäure vorhandenen mehrfach ungesättigten Fettsäuren in polymere Fettsäuren überführt, die dann bei der anschliessenden Destillation im Rückstand verbleiben. Die Polymerisation der Polyenfettsäuren kann durch einfaches Erhitzen der Fettsäuregemische auf Temperaturen oberhalb von 300°C herbeigeführt werden. In Gegenwart von Katalysatoren tritt die Polymerisation der mehrfach ungesättigten Fettsäuren bereits bei Temperaturen ab 110°C ein, wobei als Katalysatoren beispielsweise Lewis-Säuren, saure makroporöse Ionenaustauscher (DE-OS Nr. 2261067), saure Tone und Bleicherdekatalysatoren vom Montmorillonittyp (DE-OS Nr. 2335890) geeignet sind.

Bei diesen Verfahren werden jedoch nicht nur die mehrfach ungesättigten Fettsäuren in Di- und Polymerfettsäuren umgewandelt, vielmehr nimmt auch die in den Fettsäuregemischen vorhandene Ölsäure an der Polymerisation teil, wodurch die Ausbeute an nicht polymerisierten Fettsäuren gemindert wird. Führt man die Polymerisationsreaktion rein thermisch durch, so geht zudem ein Teil der Fettsäuren durch Pyrolyse verloren.

Der hauptsächliche Nachteil dieser Verfahren zur Reinigung technischer Ölsäure besteht jedoch darin, dass unter den Bedingungen der Polymerisationsreaktion eine mehr oder minder starke Isomerisierung der in den Gemischen vorhandenen Ölsäure erfolgt. Dabei bilden sich durch Wanderung der Doppelbindung iso-Ölsäuren und durch trans-Umlagerung Elaidinsäure und iso-Elaidinsäuren. Bei der im Olein gegebenenfalls vorhandenen Palmitölsäure sind analoge Reaktionen zu beobachten. Durch diese Nebenreaktionen wird der Erstarrungspunkt bzw. der Trübungspunkt des behandelten Fettsäuregemisches, der im allgemeinen bei 5 bis 10°C liegt, häufig beträchtlich erhöht. Die Aufgabe, die Qualität der technischen Ölsäure durch das Abtrennen von Linolsäure und Linolensäure zu verbessern, ist damit aber nur bedingt gelöst, weil eine wesentliche Eigenschaft des Oleins verlorengegangen ist, nämlich bei Temperaturen um 10°C noch flüssig zu sein.

Andere Verfahrensmöglichkeiten des Standes der Technik zur Abtrennung der mehrfach ungesättigten Komponenten aus dem Ausgangsmaterial sind entweder sehr aufwendig — beispielsweise die fraktionierte Kristallisation aus Aceton oder Methanol bei Temperaturen von −60°C — oder aber es fallen auch hier unerwünscht stark veränderte Reaktionsprodukte an — beispielsweise beim Versuch der selektiven Hydrierung von Linolsäure und Linolensäure zu Ölsäure.

Die Erfindung hat sich die Aufgabe gestellt, Polyensäuren und insbesondere Linolsäure und Linolensäure aus Fettsäuregemischen durch eine Polymerisationsreaktion mit anschliessender destillativer Auftrennung zu entfernen. Dabei soll keine oder keine wesentliche trans-Isomerisierung der im Gemisch vorhandenen Ölsäure- und/oder Palmitölsäurekomponenten stattfinden, so dass der Trübungspunkt des gereinigten Oleins gegenüber dem des Ausgangsmaterials praktisch unverändert ist. Aufgabe der Erfindung ist es schliesslich, ein Polymerisationsverfahren für die mehrfach ungesättigten Komponenten zu entwikkeln, das eine selektive Polymerisation dieser unerwünschten Komponenten ermöglicht und damit die bei der abschliessenden Destillation auftretenden Rückstandsverluste etwa auf die Menge der im Rohmaterial vorliegenden mehrfach ungesättigten Komponenten einschränkt.

Gegenstand der Erfindung ist dementsprechend ein Verfahren zur Abtrennung von Polyenfettsäuren aus Monoenfettsäuren enthaltenden Fettsäure- und/oder Fettsäureestergemischen durch Polymerisation der mehrfach ungesättigten Komponenten und destillative Auftrennung des Reaktionsproduktes, wobei dieses Verfahren dadurch gekennzeichnet ist, dass man vor und/oder gleichzeitig mit der Polymerisation die mehrfach ungesättigten Komponenten in Konjuenfettsäuren umwandelt, die Polymerisation durch Einleiten von Sauerstoff oder eines freien Sauerstoff einthaltenden Gases in das Fettsäuregemisch bewirkt und ein Destillationsprodukt mit einem Gehalt an mehrfach ungesättigten Komponenten < 5 Gew.% abtrennt.

Das erfindungsgemässe Verfahren sieht also grundsätzlich die drei folgenden Verfahrensstufen vor:

1. Umwandlung der im rohen Ausgangsmaterial vorhandenen mehrfach ungesättigten Fettsäuren in Konjuenfettsäuren,

2. selektive Polymerisation der Konjuenfettsäuren durch Einleiten von Sauerstoff oder eines freien Sauerstoff enthaltenden Gases in das Fettsäuregemisch, und schliesslich

3. Abtrennung der entstandenen Polymerfettsäuren durch Destillation, wobei hier die gebildeten polymeren Materialien im Destillationsrückstand verbleiben.

Das erfindungsgemässe Verfahren sieht die Möglichkeit vor, diese drei Verfahrensstufen getrennt voneinander und nacheinander vorzunehmen. In einer besonderen Ausführungsform der Erfindung ist es allerdings auch möglich, die Stufen 1 und 2 dieses Mehrstufenverfahrens in einer Verfahrensstufe zusammenzufassen.

Im folgenden werden Verfahrenseinzelheiten und bevorzugte Verfahrensbedingungen für die einzelnen Stufen des erfindungsgemässen Verfahrens geschildert, dabei wird zunächst die Variante behandelt, in der die Stufen 1 und 2 getrennt voneinander durchgeführt werden.

Die Konjugierung der mehrfach ungesättigten Komponenten erfolgt hier vorzugsweise in an sich bekannter Weise in alkalischem Medium. Sie lässt sich beispielsweise am Gemisch der freien Fettsäuren derart durchführen, dass man eine wässerige Alkaliüberschuss enthaltende Seifenlösung des Fettsäuregemisches auf Temperaturen zwischen 200 und 300° C erhitzt. Man kann aber auch Fettsäureester, insbesondere Ester mit niederen einwertigen Alkoholen — vorzugsweise ein Methylestergemisch — in Gegenwart von Alkalialkoholaten bei Temperaturen von 100 bis 200° C behandeln. Die Dauer der Konjugierungsbehandlung wird im allgemeinen so gewählt, dass die gewünschte Konjugierung zu wenigstens 85%, vorzugsweise zu 90% oder mehr, eingetreten ist. Anschliessend wird das Fettsäuregemisch aus der Seifenlösung bzw. aus dem Estergemisch durch Aussäurung bzw. durch Spalten zurückgewonnen, in dem jetzt die Linol- und Linolenfettsäure als Konjuenfettsäuren vorliegen.

In der zweiten Stufe des erfindungsgemässen Verfahrens erfolgt die Behandlung des konjugierten Fettsäuregemisches mit Sauerstoff oder einem freien Sauerstoff enthaltenden Gas — vorzugsweise mit Luft — bei einer Temperatur ab 100° C. Unter diesen Bedingungen bilden die in den Fettsäuregemischen vorhandenen Konjuenfettsäuren vermutlich über Peroxidverbindungen als Zwischenstufen höhermolekulare Verbindungen, die von den nicht polymerisierten Bestandteilen durch Destillation abtrennbar sind. Unter den erfindungsgemässen Verfahrensbedingungen der Polymerisation werden unerwünschte Isomerisierungsreaktionen z.B. eine trans-Bildung, nicht oder praktisch nicht festgestellt. Dementsprechend zeigt der durch die abschliessende Destillation abgetrennte Anteil des Reaktionsproduktes keinen oder praktisch keinen Trübungspunktanstieg.

Der Reaktionsverlauf der oxidativen Polymerisation wird hauptsächlich durch die Verfahrenstemperatur und die Reaktionsdauer bestimmt. Geeignete Verfahrenstemperaturen liegen im Bereich von 100 bis 250° C und vorzugsweise im Bereich von 120 bis 220° C. Die Verfahrensdauer liegt üblicherweise im Bereich von 0,5 bis 2 h. Die während dieser Verfahrensstufe eingesetzte Luftmenge liegt vorzugsweise im Bereich von 30 bis 60 l Luft/100 g des Einsatzmaterials.

Für die hier zunächst geschilderte Ausführungsform der Konjugierung und Polymerisation in getrennten Verfahrensstufen gelten die folgenden besonders bevorzugten Verfahrensbedingungen für die Stufe der Polymerisation: Reaktionstemperatur im Bereich von 150 bis 180° C, Reaktionsdauer 0,5 bis 1 h, Luftmenge 12 bis 17 l/h für ca. 350 g des Einsatzmaterials.

Die Luftoxydation erfolgt beispielsweise in einem von aussen beheizten cylindrischen vertikalen Reaktionsgefäss, das im Gefässboden geeignete Mittel zum Einleiten des gasförmigen freien Sauerstoff enthaltenden Behandlungsmittels aufweist. Der Zusatz von Katalysatoren wie 0,1% $KMnO_4$ oder 0,1 bis 0,3% Kobaltlinolat verbessert den Polymerisationsverlauf üblicherweise nicht.

In der abschliessenden dritten Stufe des erfindungsgemässen Verfahrens wird eine Destillation der behandelten Fettsäuregemische unter den für Fettsäuren üblichen Bedingungen durchgeführt. Die aus den konjugierten Polyenfettsäuren gebildeten Polymeren verbleiben hierbei im Destillationsrückstand. Man erhält als Destillat ein Fettsäuregemisch, das in seiner Zusammensetzung bis auf die Polyenfettsäuren dem ursprünglichen Gemisch entspricht.

Die Polyenfettsäuren sind je nach den angewandten Verfahrensbedingungen der ersten und zweiten Stufe vollständig oder weitgehend aus dem Fettsäuregemisch entfernt. Der Trübungspunkt bzw. Erstarrungspunkt des Fettsäuregemisches hat sich praktisch nicht verändert. Die Destillation liefert in der hier geschilderten Ausführungsform des erfindungsgemässen Verfahrens üblicherweise farbhelle Destillate. Die bei der Destillation anfallenden Rückstandsmengen entsprechen etwa dem Gehalt des Ausgangsmaterials an polyungesättigten Fettsäuren, so dass also tatsächlich eine praktisch selektive und quantitative Entfernung der unerwünschten Polyenfettsäuren möglich ist.

In der abgewandelten Ausführungsform des erfindungsgemässen Verfahrens werden die Reaktionen der ersten und der zweiten Stufe miteinander verbunden bzw. gleichzeitig durchgeführt. Das technische Fettsäuregemisch, dessen Polyene entfernt werden sollen, wird in dieser Ausführungsform in Gegenwart eines Konjugierungskatalysators bei erhöhten Temperaturen mit Sauerstoff bzw. dem freien Sauerstoff enthaltenden Gas behandelt. Als Katalysatoren eignen sich insbesondere Jod und/oder Jodoform. In der Regel wird hier das Gemisch der freien Fettsäuren dieser kombinierten Behandlung gemäss der ersten und zweiten Verfahrensstufe unterworfen.

Zweckmässigerweise wird der Katalysator im Ausgangsmaterial vor Beginn des Einblasens des sauerstoffhaltigen Gases gelöst. Die Lösung kann bei erhöhten Temperaturen beispielsweise bei ca. 40 bis 50° C erfolgen. Es werden nur geringe Katalysatormengen benötigt. So kann Jod beispielsweise in Mengen von 0,06 bis 0,2 Gew.% —

hier und im folgenden jeweils bezogen auf das Oleinausgangsmaterial — eingesetzt werden, üblicherweise wird mit Mengen im Bereich von 0,06 bis 0,09 Gew.% Jod gearbeitet. Jodoform kann in Mengen von 0,1 bis 0,3 Gew.% verwendet werden.

Bezüglich der sonstigen Reaktionsbedingungen gelten die zuvor gemachten allgemeinen Angaben. Als besonders günstige Reaktionsbedingungen sind für diese Ausführungsform die folgenden Verfahrensbedingungen ermittelt worden:

| Katalysator | Reaktionstemperatur | Reaktionsdauer | Luftmenge (l/h) (f. 350 g Olein) |
|---|---|---|---|
| 0,06-0,09% Jod | 180-220°C | 1-1,5 h | 12 |
| 0,1% Jodoform | 120-150°C | 0,5-1 h | 12 |

Bei der abschliessenden Destillation werden im Vergleich mit der zuvor geschilderten Ausführungsform des erfindungsgemässen Verfahrens etwas weniger farbhelle und farbstabile Oleindestillate erhalten, hauptsächlich gilt dieses für die Verwendung von Jod als Katalysator. Eine Farbverbesserung im Destillat ist durch eine Redestillation über 0,1% Borsäure möglich. Die eingesetzten Katalysatoren Jod und/oder Jodoform verbleiben bei der Aufarbeitung im wesentlichen im Destillationsrückstand.

Die Destillationsrückstände können — unabhängig von der bestimmt gewählten Ausführungsform des erfindungsgemässen Verfahrens — gewünschtenfalls in an sich bekannter Weise aufgearbeitet werden.

Das erfindungsgemässe Verfahren ist auf Fettsäure- bzw. Fettsäureestergemische beliebigen Ursprungs anwendbar. Geeignet ist es beispielsweise für alle Gemische solcher Art, deren Gehalt an höher ungesättigten zu entfernenden Komponenten im Bereich von 3 bis 25 Gew.% liegt. Es können sowohl Gemische natürlicher als auch Gemische synthetischer höherer Carbonsäuren bzw. ihrer Ester dem erfindungsgemässen Reinigungsverfahren unterworfen werden, wobei auch willkürlich zustande gekommene Gemische unter diese Definition fallen. Von besonderem Interesse ist das erfindungsgemässe Verfahren für die Reinigung technischer Ölsäure und ölsäurereicher natürlicher Fettsäuregemische. Eine besondere Vorreinigung des Ausgangsmaterials ist nicht erforderlich.

*Beispiele 1 bis 3*

Als Ausgangsmaterial wurde das Destillat einer aus tierischem Fett gewonnenen technischen Ölsäure eingesetzt. Trübungspunkt 7,0°C, JZ (nach Woburn): 94,5, Linolsäuregehalt: 10,3% und Linolensäuregehalt unter 1%. Dieses Ausgangsmaterial wird in den folgenden Beispielen 1 bis 3 in einer ersten Verfahrensstufe getrennt von der Polymerisation der Alkalikonjugierung unterworfen. Hierzu werden in einen 2-l-Rührautoklav 470 g der technischen Ölsäure und 490 g 18,5 gew.%ige wässerige Natronlauge (1,9% Alkaliüberschuss in der Gesamtmenge) gegeben. Das Reaktionsgemisch wird 4 h bei 220°C unter Rühren im Autoklaven erhitzt. Anschliessend wird der Seifenleim mit verdünnter Schwefelsäure ausgesäuert und die gewonnene Rohfettsäure mit ihrem Gehalt an konjugierten Komponenten im Vakuum destilliert.

Das Olein hatte nach der Konjugierungsreaktion einen Trübungspunkt von 7,5°C, JZ (nach Woburn): 93,9, Linolsäuregehalt: 0,5%, Gehalt an konjugierter Linolsäure: 9,7%.

Das so vorbehandelte Olein wird anschliessend unter verschiedenen Bedingungen mit Luft geblasen und dann bei einem Druck von 4 mbar destilliert. Die Verfahrensbedingungen und die dabei erhaltenen Ergebnisse sind in der folgenden Tabelle 1 zusammengefasst.

Diese Tabelle 1 gibt für die Oleindestillate nach der Behandlung mit Luft die jeweiligen Trübungspunkte, den trans-Gehalt und den Gehalt an Linolsäure und Linolensäure an.

Tabelle 1

| Bedingungen der Behandlung mit Luft | | Oleindestillate nach Behandlung mit Luft | | | |
|---|---|---|---|---|---|
| Temp. des konj. Oleins (°C) | Zeitdauer (h) | Trübungspunkt (°C) | trans-Gehalt (%) | Linolsäure | Linolensäure |
| | | | | konjugiert + nicht konjugiert Gehalt (%) | |
| Olein nach Konjugationsbehandlung | | 7,5 | 3 | 10,2 | <1 |
| *Beispiel 1* | 120 | 2 | 7,0 | 3 | 2,0 | 0 |
| *Beispiel 2* | 180 | 1 | 7,0 | 3 | 0,2 | 0 |
| *Beispiel 3* | 200 | 0,5 | 7,0 | 3 | 2,1 | 0 |

*Beispiele 4 bis 6*

In einer anderen Versuchsreihe wurde die Konjugierung der Fettsäure nicht in einer getrennten Vorstufe durchgeführt. In einem Verfahrensgang erfolgt eine katalytische Konjugation der Linol- und Linolensäure, wobei gleichzeitig durch eine Behandlung mit Luft die Konjuenfettsäuren in nicht destillierbare Polymere übergeführt wurden.

Als Ausgangsmaterial diente das gleiche Olein wie bei den Beispielen 1 bis 3.

In der folgenden Tabelle 2 sind die unterschiedlichen Bedingungen der gleichzeitigen Konjugation und Polymerisation angegeben sowie die Zusammensetzung der Oleindestillate, die nach dieser Behandlung erhalten worden sind.

Tabelle 2

| Bedingungen der Behandlung mit Luft | | | Oleindestillate nach Behandlung mit Luft | | | |
|---|---|---|---|---|---|---|
| | | | | | Linolsäure | Linolensäure |
| Temp. des Oleins (°C) | Katalysator-zusatz | Zeitdauer (h) | Trübungs-punkt (°C) | trans-Gehalt (%) | konjugiert + nicht konjugiert Gehalt (%) | |
| Olein nicht vorbehandelt | | | 7,0 | 3 | 10,8 | <1 |
| *Beispiel 4* 120°C | 0,1% Jodoform | 0,5 | 7,0 | 4 | 1,1 | 0 |
| *Beispiel 5* 180°C | 0,1% Jodoform | 1,0 | 8,0 | 4 | 5,6 | 0 |
| *Beispiel 6* 220°C | 0,06% Jod | 1,0 | 7,6 | 4 | 3,2 | 0 |

**Patentansprüche**

1. Verfahren zur Abtrennung von Polyenfettsäuren aus Monoenfettsäuren enthaltenden Fettsäure- und/oder Fettsäureestergemischen durch Polymerisation der mehrfach ungesättigten Komponenten und destillative Auftrennung des Reaktionsproduktes, dadurch gekennzeichnet, dass man vor und/oder gleichzeitig mit der Polymerisation die mehrfach ungesättigten Komponenten in Konjuenfettsäuren umwandelt, die Polymerisation durch Einleiten von Sauerstoff oder eines freien Sauerstoff enthaltenden Gases in das Fettsäuregemisch bewirkt und ein Destillationsprodukt mit einem Gehalt an mehrfach ungesättigten Komponenten < 5 Gew.% abtrennt.

2. Verfahren nach Anspruch 1, dadurch gekennzeichnet, dass man die Konjugierung der mehrfach ungesättigten Komponenten als getrennten Verfahrensschritt vor der Polymerisation in alkalischem Medium durchführt.

3. Verfahren nach Anspruch 1, dadurch gekennzeichnet, dass man die Konjugierung der mehrfach ungesättigten Komponenten gleichzeitig mit der Polymerisation unter Zusatz von Jod und/oder Jodoform als Katalysatoren durchführt.

4. Verfahren nach den Ansprüchen 1 bis 3, dadurch gekennzeichnet, dass man zur Polymerisation Luft einsetzt und dabei das Blasen mit dem Luftstrom im Temperaturbereich von 100 bis 250°C, vorzugsweise im Bereich von 120 bis 220°C für einen Zeitraum von 0,5 bis 2 h durchführt.

5. Verfahren nach Anspruch 4, dadurch gekennzeichnet, dass man 30 bis 60 l Luft/1000 g Fettsäuregemisch einsetzt.

6. Verfahren nach Ansprüchen 1 bis 5, dadurch gekennzeichnet, dass man durch Destillation ein Monoenfettsäuren enthaltendes Reaktionsprodukt abtrennt, das im Vergleich mit dem Ausgangsmaterial einen praktisch unveränderten Trübungspunkt und Gehalt an trans-Verbindungen aufweist und dessen Gehalt an mehrfach ungesättigten Komponenten, vorzugsweise < 3 Gew.%, liegt.

7. Verfahren nach Ansprüchen 1 bis 6, dadurch gekennzeichnet, dass man technische Ölsäure dem Verfahren unterwirft.

**Claims**

1. A process for separating polyene fatty acids from fatty acid and/or fatty acid ester mixtures containing monoene fatty acids by polymerizing the polyunsaturated components and working up the reaction product by distillation, characterized in that the polyunsaturated components are converted into conjuene fatty acids before and/or during polymerization, in that polymerization is carried out by introducing oxygen or a gas containing free oxygen into the fatty acid mixture, and in that a distillation product containing less than 5% by weight of polyunsaturated components is separated off.

2. A process as claimed in claim 1, characterized in that conjugation of the polyunsaturated components is carried out as a separate process step before polymerization in an alkaline medium.

3. A process as claimed in claim 1, characterized in that conjugation of the polyunsaturated components is carried out during polymerization in the presence of added iodine and/or iodoform as catalysts.

4. A process as claimed in claims 1 to 3, characterized in that air is used for polymerization and in that the stream of air is introduced for 30 min to 2 h at temperatures in the range from 100 to 250°C and preferably at temperatures in the range from 120 to 220°C.

5. A process as claimed in claim 4, characterized in that the air is used in a quantity of 30 to 60 l/1000 g of fatty acid mixture.

6. A process as claimed in claims 1 to 5, characterized in that a reaction product containing monoenefatty acids which, compared with the starting material, has substantially the same cloud point and content of transcompounds and which preferably contains less than 3% by weight of polyunsaturated components, is separated off by distillation.

7. A process as claimed in claims 1 to 6, characterized in that technical oleic acid is subjected to the process.

## Revendications

1. Procédé pour séparer les acides gras polyéniques de mélanges d'acides gras et/ou d'esters d'acides gras contenant des acides gras monoéniques par polymérisation des composants polyinsaturés et par séparation du produit de réaction par distillation, caractérisé en ce que, avant et/ou durant la polymérisation, on convertit les composants polyinsaturés en acides gras polyéniques conjugués, on provoque la polymérisation par injection d'oxygène ou d'un gaz contenant de l'oxygène libre dans le mélange d'acides gras et on sépare un produit de distillation contenant moins de 5% en poids de composants polyinsaturés.

2. Procédé selon la revendication 1, caractérisé en ce que la conjugaison des composants polyinsaturés est réalisée en stade opératoire séparé avant la polymérisation en milieu alcalin.

3. Procédé selon la revendication 1, caractérisé en ce que la conjugaison des composants polyinsaturés est effectuée en même temps que la polymérisation avec addition d'iode et/ou d'iodoforme en tant que catalyseurs.

4. Procédé selon les revendications 1 à 3, caractérisé en ce que l'on utilise l'air pour provoquer la polymérisation et en ce que l'insufflation du courant d'air est réalisée dans un intervalle de température de 100 à 250°C, de préférence de 120 à 220°C, pendant une durée de 0,5 à 2 h.

5. Procédé selon la revendication 4, caractérisé en ce que l'on utilise de 30 à 60 l d'air/1000 g du mélange d'acides gras.

6. Procédé selon les revendications 1 à 5, caractérisé en ce que l'on sépare par distillation un produit de réaction contenant les acides gras monoéniques et qui, comparativement au produit de départ, a un point de trouble et une teneur en composés trans pratiquement non modifiés et une teneur en composant polyinsaturés qui est de préférence inférieure à 3% en poids.

7. Procédé selon les revendications 1 à 6, caractérisé en ce que l'on traite par le procédé de l'acide oléique technique.